# EUROPÄISCHE PATENTSCHRIFT

(11) **EP 3 651 842 B1**
(45) Veröffentlichungstag und Bekanntmachung des Hinweises auf die Patenterteilung: **23.03.2022**
(21) Anmeldenummer: 18756367.1
(22) Anmeldetag: 11.07.2018
(51) Int. Cl.: A61M 16/00, A61M 16/04

(54) **SYSTEM UND HERSTELLVERFAHREN ZUR UNTERSTÜTZUNG DES PULMONALEN GASAUSTAUSCHS BEI PATIENTEN**
SYSTEM AND MANUFACTURING METHOD FOR ASSISTING PULMONARY GAS EXCHANGE FOR PATIENTS
SYSTÈME ET MÉTHODE DE FABRICATION POUR AIDER L'ECHANGE GAZEUX PULMONAIRE POUR LES PATIENTS

(30) Priorität: 13.07.2017 DE 102017006655
(43) Veröffentlichungstag der Anmeldung: 20.05.2020
(60) Teilanmeldung: 22000029.3
(73) Patentinhaber: Gründler GmbH, 72250 Freudenstadt (DE)
(72) Erfinder: GRÜNDLER, Markus, 72250 Freudenstadt (DE); GRÜNDLER, Christoph, 72250 Freudenstadt (DE); RESLO, Bernd, 86934 Reichling (DE); JOOST, Thilo, 61169 Friedberg (Hessen) (DE); KÖBRICH, Rainer, 68809 Neulussheim (DE)
(86) Internationale Anmeldenummer: PCT/DE2018/000215
(87) Internationale Veröffentlichungsnummer: WO 2019/011365

(56) Entgegenhaltungen:
- EP-A1- 1 329 238
- EP-A1- 1 459 778

## Beschreibung

Die Erfindung betrifft ein System zur Unterstützung des pulmonalen Gasaustauschs bei Patienten mit den Merkmalen des Anspruchs 1 sowie ein Verfahrens zur Herstellung des Systems unter Verwendung einen Patientensets mit den Merkmalen des Anspruchs 10.

Bei Atemstörungen ist die künstliche Beatmung seit Langem etabliert. Hierbei wird üblicherweise zyklisch das Atemgas mittels Überdruck über einen in die Luftröhre eingeführten Schlauch oder mittels Gesichtsmaske in die Atemwege appliziert, während die Ausatmung durch die passiven Rückstellkräfte des Atemapparates automatisch dann stattfindet, wenn der externe Überdruck abgebaut wird. Um Schädigungen durch hohe Beatmungsdrücke zu vermeiden, wird versucht, das Atemzugvolumen und die Beatmungsdrücke unter Beatmung möglichst klein zu halten. Beschränkend wirkt sich dabei stets das sogenannte "Pendelvolumen" oder "Totraumvolumen" aus, das im luftleitenden System von Patient und Beatmungssystem bidirektional bewegt wird. Das im Pendelvolumen verbleibende ausgeatmete Volumen wird beim darauffolgenden Atemzug erneut eingeatmet, der Anteil von Frischluft nimmt also mit zunehmendem Pendelvolumenanteil ab. Je größer das Totraumvolumen im Verhältnis zum Atemzugvolumen ist, desto ineffektiver ist der Gasaustausch.

Aus der Druckschrift DE 60 2004 003 409 T2 ist ein System bekannt, das als Unterstützungssystem mit einem Beatmungssystem gekoppelt ist. Das Beatmungssystem weist eine Patientenleitung auf, die vom Y-Stück in die Luftröhre eines Patienten reicht. Durch eine mit der Patientenleitung verbundene Aspirationsleitung des Unterstützungssystems wird während der Endphase der Ausatmung aus der Patientenleitung ausgeatmetes Gas abgesaugt. Gleichzeitig wird an einer anderen, näher am Ventilator gelegenen Stelle der Inspirationsleitung durch eine weitere Leitung frisches Gas nachgeführt, um die Funktion des Beatmungssystems nicht zu stören. Damit wird das durch die Patientenleitung gebildete Totraumvolumen von ausgeatmeter Luft teilweise befreit. Beim darauffolgenden Einatmen wird somit weniger ausgeatmetes Gas erneut eingeatmet, sondern eher frisches Gas. Das abgesaugte Gas wird zu Beginn der nächsten Ausatmung wieder der Patientenleitung zugeführt.

Allerdings ist dieses bekannte System nur eng gekoppelt an ein Beatmungssystem betreibbar, da die Steuerung des Unterstützungssystems auf Basis der Signale des Beatmungssystems erfolgt, insbesondere Signale zum Strömungs-Zeit-Verhältnis. Da es keine standardisierten Schnittstellen für derartige Signale gibt, muss zwischen dem Unterstützungssystem und dem Beatmungssystem jeweils eine spezifische Schnittstelle geschaffen werden. Weiterhin ist das bekannte System empfindlich gegen Verstopfungen der Aspirationsleitung, so dass eine zuverlässige Unterstützung des Beatmungssystems nicht gewährleistet ist.

Aus der EP 1 329 238 und EP 1 459 778 sind Systeme zur Unterstützung des pulmonalen Gasaustauschs bei Patienten und zur Kopplung an ein Beatmungssystem bekannt. Diese Dokumente offenbaren Pumpeinheiten die mechanisch fest miteinander gekoppelt sind und somit nicht voneinander unabhängig gesteuert werden können.

Der Erfindung liegt daher die Aufgabe zugrunde, ein System zur Unterstützung des pulmonalen Gasaustauschs bei Patienten zu schaffen.

Diese Aufgabe wird erfindungsgemäß durch das System mit den Merkmalen des Anspruchs 1 beziehungsweise durch das Verfahren gemäß Anspruch 10 zur Herstellung des Systems unter Verwendung eines Patientensets gelöst. Das erfindungsgemäße System dient der Unterstützung des pulmonalen Gasaustauschs bei Patienten und zur Kopplung an ein Beatmungssystem Das System weist einen flexiblen Schlauch auf, der in die Luftröhre eines Patienten einführbar ist, insbesondere bis in die distale Trachea bzw. die Hauptbronchien. Dabei kann der Schlauch durch einen operativ geschaffenen Zugang, insbesondere im Halsbereich, oder durch Mund oder Nase in die Luftröhre eingeführt sein. Insbesondere im Fall des durch ein Beatmungssystem beatmeten Patienten kann der flexible Schlauch durch einen Endotrachealtubus oder eine Trachealkanüle des Beatmungssystems in die Luftröhre eingeführt oder in den Endotrachealtubus beziehungsweise die Trachealkanüle integriert sein. Weiterhin weist das System eine Pumpeinheit und eine Reservoireinheit auf. Die Pumpeinheit dient dazu, Gas aus der Lunge, aus der Luftröhre oder aus dem Leitungssystem eines Beatmungsgerätes abzusaugen und wieder durch Pumpen zurück zu führen, was nachfolgend auch als "Rückführung" bezeichnet wird. Die Reservoireinheit dient der Zwischenspeicherung des abgesaugten Gases, bevor es wieder zurückgeführt wird. Die Zwischenspeicherung kann außerdem zur Temperierung des Gases genutzt werden. Die Pumpeinheit und die Reservoireinheit bilden vorzugsweise eine gemeinsame Einheit und sind insbesondere als Kolbenpumpe ausgeführt. Weiterhin weist das System eine Steuerung derart auf, dass über den flexiblen Schlauch mittels der Pumpeinheit eine Absaugung und eine Rückführung des abgesaugten Gases einstellbar ist. Die Absaugung erfolgt exspiratorisch und insbesondere end-exspiratorisch, während die Rückführung insbesondere spät inspiratorisch erfolgt. Dies ermöglicht den oben beschriebenen Effekt der Minderung des Totraumvolumens, da bei der Einatmung der Rückatemanteil verbrauchter Atemgase reduziert wird. In der Folge wird eine Vergrößerung der Partialdruckdifferenzen zwischen Blut- und Gasseite in der Lunge erreicht, was zu einer Verbesserung der Oxygenierung sowie zu einer erhöhten Eliminierung lungengängiger Stoffe, z.B. CO₂ oder Alkohol, führt. "Rückführung" meint eine zumindest teilweise Zurückleitung in den Patienten und zwar insbesondere durch den flexiblen Schlauch hindurch, durch den zuvor die Absaugung erfolgt ist.

Das System ist dadurch gekennzeichnet, dass es einen Sensor, insbesondere einen Sensor zur Bestimmung des Drucks, der Strömungsgeschwindigkeit und/oder der Zusammensetzung eines Gases, aufweist. Durch den Sensor können auf einfache Weise Daten über den Zustand des Systems oder des Patienten bestimmt werden und hierdurch insbesondere Veränderungen auf der Patientenseite, wie beispielsweise eine Änderung des Atemrhythmus und/oder Störungen, wie eine Verstopfung des flexiblen Schlauchs, identifiziert werden. Eine Signalaufnahme eines Sensors am oder im Patient oder auch im Bereich des flexiblen Schlauchs kann dazu dienen, Informationen über die Tätigkeit eines Beatmungssystems, mit dem das System pneumatisch gekoppelt ist, zu erhalten, so dass keine Daten-Schnittstelle zwischen den beiden Systemen notwendig ist. Die Daten des Sensors können zur Anpassung an den Patienten und/oder an das Beatmungsgerät dienen oder Basis für eine Behebung einer Störung sein, sei es durch einen Bediener oder durch das System selbst. Insbesondere weist das System eine Einrichtung zum Entgegenwirken einer Verstopfung im flexiblen Schlauch in Abhängigkeit von Daten des Sensors auf.

Vorzugsweise ist der Sensor bezüglich der Strömung des vom System geführten Gases zwischen einem offenen, patientenseitigen Ende des flexiblen Schlauchs und der Reservoireinheit angeordnet. Alternativ könnte der Sensor in der Reservoireinheit selbst angeordnet sein, was jedoch einen aufwendigeren Aufbau zur Folge hat. "Zwischen" meint hier nicht, dass der Sensor selbst durchflossen sein muss, sondern er kann auch an einer Abzweigung angeordnet sein.

Um Verstopfungen im flexiblen Schlauch sowie ein Festsaugen zu minimieren, schlägt die Erfindung vor, diesen flexiblen Schlauch an seinem patientenseitigen offenen Ende vorzugsweise mit mehreren auf dem Umfang angeordneten seitlichen Öffnungen auszustatten - vergleichbar mit atraumatischen Absaugkathetern. Vorzugsweise sind diese Löcher durch kleine Vorsprünge / Abweiser geometrisch vor hochgehustetem Sekret etc. geschützt.

Um Verstopfungen im System, insbesondere im flexiblen Schlauch, feststellen zu können, schlägt die Erfindung vor, dass der Sensor ein Hochdrucksensor und derart gestaltet und angeordnet ist, dass er zur Erfassung der durch die Pumpeinheit erzeugten Druckschwankungen geeignet ist. Über die Amplitude und Form der Druckschwankungen kann auf eine Verstopfung oder auch eine Leckage zurück-geschlossen werden, nämlich beim Einsaugen durch einen signifikant niedrigeren Druck oder einen langsameren Druckausgleich als bei früheren Einsaugvorgängen und beim Zurückführen durch einen signifikant höheren Druck oder einen langsameren Druckausgleich. Ein Hochdrucksensor ist insbesondere dann geeignet, wenn er eine für die genannte Funktion ausreichende zeitliche Auflösung, einen ausreichenden Messbereich und eine ausreichende Messgenauigkeit in dem relevanten Druckbereich aufweist. Insbesondere beträgt die Auflösung 1 mbar oder weniger innerhalb eines Druckbereichs von mindestens +/- 750 mbar, bei einer zeitlichen Auflösung von mindestens 25 Messwerten pro Sekunde.

Vorzugsweise weist das System einen Feindrucksensor auf zur Erfassung der durch Spontanatemaktivitäten des Patienten erzeugten Druckschwankungen im flexiblen Schlauch. Der Feindrucksensor kann der oben genannte Sensor sein, insbesondere ist er jedoch ein zusätzlicher Sensor. Insbesondere gibt es also einen Hochdrucksensor und einen Feindrucksensor. Der Feindrucksensor ermöglicht den Einsatz des Systems bei einem nicht-beatmeten Patienten. Solange die Pumpeinheit nicht arbeitet, stellt sich in dem flexiblen Schlauch im Wesentlichen der gleiche Druck wie in der Luftröhre des Patienten ein. Aus den Daten des Feindrucksensors kann somit auf die Atemaktivität des Patienten zurück geschlossen werden, also erfasst werden, an welcher Stelle des Atemzyklus sich der Patient aktuell befindet und mit welcher Intensität die Atmung erfolgt. Der Feindrucksensor ist hierfür dann geeignet, wenn er eine für die genannte Funktion ausreichende zeitliche Auflösung, einen ausreichenden Messbereich und eine ausreichende Messgenauigkeit in dem relevanten Druckbereich aufweist. Insbesondere beträgt die Auflösung 0,1 mbar oder weniger innerhalb eines Druckbereichs von mindestens +/- 5 mbar, bei einer zeitlichen Auflösung von mindestens 25 Messwerten pro Sekunde. Sofern der Feindrucksensor durch höhere Drücke, wie sie beispielsweise beim Husten und Sprechen auftreten können, beschädigt werden könnte, ist er in geeigneter Weise zu schützen, vorzugsweise durch ein Sensor-Schutzventil. Dadurch dass mit dem Feindrucksensor die Atemaktivität des Patienten erfasst werden kann, ist das System ohne weitere Geräte zur Unterstützung der Atmung einsetzbar, also insbesondere auch für Patienten, die nicht durch ein Beatmungssystem beatmet werden. Die Unterstützung der Spontanatmung ist durch die Verringerung des oben beschriebenen negativen Effekts des Totraumvolumens eine sehr effektive Hilfe bei einer Ateminsuffizienz. Der Einsatz des Systems ist dabei aber vor allem schonender als eine Beatmung mittels Beatmungssystem, die unnatürliche und potentiell schädliche Druckverhältnisse in den Atemwegen erzeugen kann. Solange der Patient über eine Spontanatmung verfügt, kann durch den Einsatz des Systems gegebenenfalls auf eskalierende Therapieverfahren verzichtet werden, wie beispielsweise eine nicht-invasive oder invasive Beatmung und/oder ein extrakorporales Gasaustauschverfahren (künstliche Lunge oder Herz-Lungen-Maschine). Gleichzeitig steht das System nicht in Widerspruch zu einer Beatmung mittels Beatmungssystem, sondern es kann im Zusammenspiel mit dem Beatmungssystem oder statt diesem betrieben werden.

Vorzugsweise weist das System eine zuschaltbare Zuleitung auf, durch die statt oder zusätzlich zu dem rückgeführten Gas ein Volumenstrom eines weiteren Gases zu dem flexiblen Schlauch geführt werden kann. "Zuschaltbar" meint hier, dass eine strömungstechnische Verbindung geöffnet und geschlossen werden kann. Die Zuführung eines weiteren Gases erweitert die therapeutischen Behandlungsmöglichkeiten durch das System. "Weiteres Gas" meint hier reine Gase, wie beispielsweise Sauerstoff, Helium, NO oder CO₂, aber auch Gemische hieraus, beispielsweise Luft. Insbesondere kann durch die gezielte Zuführung von Sauerstoff die Oxygenierung erheblich unterstützt werden. Die Zuführung weiterer Gase erfolgt insbesondere in Abhängigkeit der Partialdrücke im Blut oder weiterer Messwerte wie der Sauerstoffsättigung, welche durch weitere Sensoren erfasst werden können, insbesondere durch solche, die nicht Teil des Systems sind und über eine Schnittstelle gekoppelt sind. Durch die Zufuhr von CO₂ ist insbesondere eine isokapnische Hyperventilation möglich, ein etabliertes Verfahren beispielsweise zur Therapie einer Kohlenmonoxidvergiftung.

Der flexible Schlauch weist vorzugsweise einen ersten Schlauchabschnitt zum Einführen in die Luftröhre und einen zweiten Schlauchabschnitt zur Verbindung des ersten Schlauchabschnitts mit der Reservoireinheit mit einem größeren Außenquerschnitt auf. Durch den dünneren, ersten Schlauchabschnitt kann eine patientenschonende Absaugung aus oder Einleitung in die Luftröhre erfolgen bei durch den geringen Durchmesser nur minimal gestörten Strömungsverhältnissen und der zweite Schlauchabschnitt erlaubt einen größeren Innenquerschnitt und damit einen Gastransport mit geringeren, leitungsbedingten Druckverlusten.

Um das System bei einem beatmeten Patienten zur Unterstützung des Beatmungssystems betreiben zu können, schlägt die Erfindung vor, dass zur Absaugung und Rückführung von Gas aus dem Leitungssystem des Beatmungssystems ein weiterer flexibler Schlauch, eine weitere Pumpeinheit und eine weitere Reservoireinheit vorgesehen werden. Es bestehen somit zwei Stränge aus jeweils einem flexiblen Schlauch, einer Pumpeinheit und einer Reservoireinheit. Beide Stränge werden insbesondere von der Steuerung gesteuert. Ein Strang kann wie oben beschrieben Atemluft aus der Luftröhre absaugen und dieses Gas wieder zumindest teilweise dorthin zurückführen und ein anderer Strang kann aus dem Leitungssystem des Beatmungssystems Gas absaugen und wieder zurückführen. Insbesondere erfolgt die Steuerung der beiden Stränge so, dass im Wesentlichen jeweils der eine Strang saugt, während der andere zurückführt. Dies hat den erheblichen Vorteil, dass es im Bereich des Beatmungssystems zu keinen störenden Veränderungen der Druck- und Flussverläufe durch das System kommt. Für das Beatmungssystem bleibt das erfindungsgemäße System somit "unsichtbar" und das Beatmungssystem kann ohne Anpassungen betrieben werden.

Vorzugsweise ist eine separate Sensorleitung vorgesehen, die pneumatisch im Bereich des Y-Stücks des Beatmungsgerätes eingekoppelt ist. Hierdurch kann zu jedem Zeitpunkt Status und Aktivität des Beatmungsgerätes im Verhältnis zum Patienten verfolgt und in der Steuerung berücksichtigt werden. Diese Sensorleitung kann aus mehreren, voneinander getrennten Lumina bestehen und durch Anordnung auf unterschiedlichen Seiten eines Strömungshindernisses im Gasstrom Aussagen über Höhe, Richtung und zeitlichen Verlauf von Gasflüssen ermöglichen.

Erfindungsgemäß sind die beiden Pumpeinheiten unabhängig voneinander durch die Steuerung steuerbar. Insbesondere sind sie nicht mechanisch starr gekoppelt. Durch die unabhängige Ansteuerung kann die Trägheit des Gesamtsystems aus Beatmungssystem und System, die sich insbesondere aus der Kompressibilität des Gases und den Strömungswiderständen ergibt, ausgeglichen werden. Insbesondere erfolgt das Pumpen und Ansaugen leicht zeitversetzt und mit unterschiedlichen Geschwindigkeitsverläufen. Hierdurch kann insbesondere vermieden werden, dass das Ansaugen im lungennahen Strang zu störenden Druckänderungen im Leitungssystem des Beatmungssystems führt, indem beispielsweise mit einem zeitlichen Vorlauf oder einer höheren Geschwindigkeit Gas durch den anderen Strang eingepumpt wird. Ein Unterdruck im Leitungssystem während dieser end-exspiratorischen Totraumabsaugung beziehungsweise ein daraus resultierender Gasfluss aus dem Beatmungssystem Richtung Patient könnte dem Beatmungssystem ein Einatmen suggerieren, was die Funktion des Beatmungssystems stören würde.

In einer bevorzugten Ausführungsform weist das System einen Gasanalyse-Sensor, insbesondere einen CO₂-Sensor, auf. Dies erlaubt eine permanente Überwachung der Effizienz des Systems sowie des Zustands des Patienten, da mit jedem Absaugvorgang eine Analyse des ausgeatmeten Gases erfolgen kann. Diese Überwachung kann einerseits zur Steuerung des Systems und andererseits zur Anzeige des Patientenzustands für einen behandelnden Therapeuten dienen. Der Gasanalyse-Sensor ist bezüglich der Strömung des abgesaugten Gases zwischen einem offenen, patientenseitigen Ende des flexiblen Schlauches und der Reservoireinheit angeordnet.

Durch das Absaugen, Zwischenspeichern und Rückführen des Atemgases kann es zu einer nicht gewünschten Temperaturänderung kommen. Die Erfindung schlägt daher vor, dass der flexible Schlauch und/oder die Reservoireinheit mit einer thermischen Isolierung umhüllt ist und/oder eine Temperiereinheit aufweist. Die Temperiereinheit ist insbesondere eine Heizeinheit.

Wie erwähnt kann eine Verstopfung der gasführenden Komponenten, insbesondere des flexiblen Schlauches, durch körpereigene Sekrete des Patienten und dergleichen, nicht ausgeschlossen werden. Der Sensor, insbesondere der oben genannte Hochdrucksensor, kann einer Detektion einer solchen Störung dienen. Um dieser Störung, insbesondere automatisch, entgegen wirken zu können, schlägt die Erfindung vor, dass das System eine Verzweigung mit einer zuschaltbaren Öffnung zur Umwelt aufweist, derart dass mittels der Pumpeinheit aus der Umgebung Luft angesaugt werden kann zum Freiblasen des flexiblen Schlauchs. Die Öffnung zur Umwelt kann mit einem Filter gegen Eindringen von Keimen und Verschmutzungen geschützt sein. Die zuschaltbare Öffnung kann alternativ auch dazu genutzt werden, Beatmungsgase anstelle der Umgebungsluft zuzuschalten, wie dies oben für die zuschaltbare Zuleitung beschrieben ist. Ebenso kann die zuschaltbare Öffnung auch als Notentlüftung dienen für den Fall eines unerwünschten Überdrucks.

Um eine isokapnische Hyperventilation zu unterstützen, ist beispielsweise denkbar, die zuschaltbare Öffnung des zweiten Strangs mit einer CO₂-Druckgasquelle zu verbinden.

Vorzugsweise ist die Pumpeinheit planmäßig durch den Anwender werkzeuglos koppelbar und trennbar von einer Antriebseinheit für die Pumpeinheit. Dies ermöglicht es, die Pumpeinheit beim Wechsel von einem Patienten zum nächsten aufzubereiten oder durch eine frische zu ersetzen, während die Antriebseinheit unmittelbar wiederverwendet werden kann. Durch die werkzeuglose Koppelbarkeit ist insbesondere ein schneller, einfacher Wechsel der Pumpeinheit möglich.

Die Erfindung schlägt außerdem vor, dass das System eine Kommunikationsschnittstelle für den kabelgebundenen oder drahtlosen Austausch von Informationen mit Drittgeräten, externen Sensoren und/oder Fernüberwachungssystemen aufweist. Beispielsweise kann ein weiterer Sensor, wie ein transcutaner CO₂-Sensor und/oder eine Blutgasanalyseeinrichtung, entweder in die Steuerung einbezogen oder eine ohnehin vorhandene Ausgabeeinheit der Steuerung zur Anzeige der Messwerte des weiteren Sensors genutzt werden.

Vorzugsweise weist das System eine Durchführungstülle auf zum Durchführen und Fixieren des flexiblen Schlauchs insbesondere beim spontanatmenden nicht beatmeten Patienten. Die Durchführungshülle kann nach operativer Schaffung einer Öffnung von außen in die Luftröhre in diese Öffnung eingesetzt werden, um die Öffnung offen zu halten, auch wenn der flexible Schlauch nicht durch diese Durchführungstülle gelegt ist. Sollte der Patient das System nicht benötigen oder zwischenzeitlich über ein Beatmungssystem beatmet werden, so wächst die Öffnung nicht zu, sondern wird von der Durchführungstülle offen gehalten. Die Durchführungstülle ist insbesondere ringförmig. Vorzugsweise weist das System einen Stopfen zum Schließen der Durchführungstülle auf, wenn kein flexibler Schlauch durch sie geführt ist. Des Weiteren weist die Durchführungstülle insbesondere eine Klemmvorrichtung auf, um den eingeführten flexiblen Schlauch in der gewünschten Position gegen Verrutschen zu sichern. Eine Lagekontrolle wird durch aufgebrachte Markierungen und durch Sichtbarkeit in bildgebenden Verfahren (z.B. Röntgen) ermöglicht.

Die Steuerung ist vorzugsweise so gestaltet, dass in Abhängigkeit von Daten des Sensors und/oder Eingaben über eine Benutzerschnittstelle eine Regelung und/oder Ausgabe mit mindestens einer der folgenden Ausgangsgrößen erfolgt: Zeitpunkt des Pumpens und/oder Absaugens, Geschwindigkeitsprofil des Pumpens und/oder Absaugens, Volumen des Pumpens und/oder Absaugens. Ziel hierbei ist immer eine nicht vorhandene oder minimierte Störung der Atmung/Beatmung des Patienten. Dabei ist mit "Regelung" gemeint, dass ein Regelkreis gebildet wird, während mit "Ausgeben" gemeint ist, dass ein reines Steuern, ohne Bildung eines Regelkreises, erfolgt.

Die Bestimmung des Beginns der Absaugung erfolgt insbesondere in Abhängigkeit von dem gemessenen Beginn der Exspiration und dem erwarteten Ende der Exspiration. Der Beginn der Exspiration kann durch die Messung eines signifikanten Abfalls des Drucks im Bereich des flexiblen Schlauchs zwischen Absaugen und Pumpen, oder durch einen separaten Sensor, ermittelt werden. Alternativ oder zusätzlich kann die Bestimmung des Beginns der Absaugung durch Analyse des zeitlichen Verlaufs des Drucks, also der Druckänderung, im Bereich des flexiblen Schlauchs erfolgen. Beim Ausatmen fällt der Druck, das heißt die Druckänderung ist hier ein Druckabfall, wobei der Druckabfall pro Zeiteinheit immer geringer wird, das heißt die erste Ableitung des Drucks nach der Zeit ist negativ und die zweite Ableitung positiv. Der Beginn der Absaugung kann durch Orientierung an einem Schwellwert für den Druckabfall (erste Ableitung des Drucks nach der Zeit) erfolgen. Das Absaugen wird gegebenenfalls nach einer definierten Verzögerung gestartet, wenn der Druckabfall unter einen Schwellwert sinkt, was gleichbedeutend ist mit dem Überschreiten eines bestimmten Werts der ersten Ableitung des Drucks nach der Zeit.

Ein weiteres Signal, das für die Zeitsteuerung verwendet werden kann, ist die Druckdifferenz zwischen verschiedenen Messpunkten und deren Änderungsrate im zeitlichen Verlauf. So ist beispielsweise ein höherer Druck im flexiblen Schlauch des Strangs an der Luftröhre gegenüber dem Druck im flexiblen Schlauch des Strangs am Leitungssystem des Beatmungsgeräts ein Maß für den Ausatemfluss. Die Injektion des verbrauchten Gases erfolgt entsprechend dann, wenn im Wesentlichen kein Gasfluss also kein Druckgradient mehr Richtung Patient besteht oder dieser Gasfluss gerade eben umkehrt als Zeichen der beginnenden Exspiration.

Vorzugsweise weist die Steuerung ein Programm zur Optimierung des Absaugzeitpunkts auf, wobei damit der Start der Absaugung gemeint ist. Dabei ist das Ziel eine möglichst späte Absaugung innerhalb der Exspiration, da in dieser Phase die CO₂-Konzentration maximal und damit die Methodik der Totraumabsaugung am effektivsten ist. Wie gut dies erreicht wird, beschreibt die sogenannte "Synchronisation". Allerdings muss die Absaugung vorzeitig abgebrochen werden, wenn der Patient früher als vom System erwartet, also noch während der Absaugung, einatmet. Das Programm zur Optimierung stellt eine Häufung von derartigen Abbrüchen fest und passt gegebenenfalls den Absaugzeitpunkt an, in dem dieser vorverlegt wird, was auch schrittweise erfolgen kann. Die Häufung von Abbrüchen bedeutet eine schlechte Synchronisation. Umgekehrt kann das Programm eine Verlegung des Absaugzeitpunkts zeitlich nach hinten bewirken, wenn es zu keinen Abbrüchen kommt, um eine noch spätere Absaugung zu erreichen.

Alternativ oder zusätzlich zum Programm zur Optimierung des Absaugzeitpunkts weist das System vorzugsweise ein Programm zur Optimierung der Absauggeschwindigkeit auf. So kann bei einer schlechten Synchronisation alternativ oder zusätzlich zu einer Verschiebung des Absaugzeitpunkts die Absauggeschwindigkeit erhöht und bei einer besonders guten Synchronisation verringert werden.

Insbesondere ist das System so gestaltet, dass es den Synchronisationsverlauf, also die Zahl der Abbrüche pro Zeiteinheit aufgrund einer vorzeitigen Einatmung des Patienten, visualisiert.

Vorzugsweise weist die Steuerung ein Programm für den Betrieb beim beatmeten Patienten auf, derart dass aufgrund von Daten des Sensors auf eine zunehmende Sekretansammlung in der Lunge geschlossen und in der Folge mittels einer Bedienerschnittstelle, insbesondere einem Bildschirm oder einer Kontrollleuchte, ein Signal für die Empfehlung einer trachealen Absaugung gegeben wird. Beim beatmeten Patienten ist die Sekretbildung in den Atemwegen dahingehend eine Herausforderung, dass das Sekret zur Vermeidung einer Verstopfung der Atemwege / Lunge immer wieder abgesaugt werden muss, wobei ein hierfür passender Zeitpunkt nicht einfach bestimmbar ist. Eine zunehmende Sekretansammlung kann vom erfindungsgemäßen System beispielsweise dadurch festgestellt werden, dass sich von Zyklus zu Zyklus bei gleicher Pumpaktivität die Druckverläufe im Bereich des flexiblen Schlauchs ändern, weil sich durch die Sekretansammlung eine Verstopfung und damit eine Drosselwirkung im flexiblen Schlauch ergibt. Dabei ist mit dem "Zyklus" der Atemzyklus des Patienten gemeint. Die Erfassung der Druckverläufe erfolgt insbesondere mit dem oben genannten Hochdrucksensor. Das Programm kann insbesondere so gestaltet sein, dass das Signal für eine tracheale Absaugempfehlung erst gegeben wird, wenn eine bestimmte Anzahl bzw. Häufigkeit von Versuchen zum Freiblasen des flexiblen Schlauchs wie oben beschrieben stattfand.

In einer bevorzugten Ausführungsform ist das System derart gestaltet, dass durch die Pumpeinheit ein Gasstrom mit unstetem, insbesondere gepulsten, Druckverlauf am patientenseitigen Endes des flexiblen Schlauchs erzeugbar ist. Hierdurch kann ein unsteter Gasstrom in die Lunge eines Patienten gefördert werden, was einerseits zur Verbesserung des Gasaustausches in der Lunge dienen kann und andererseits der Mobilisierung von Sekret in den Atemwegen.

Insbesondere ist das System derart gestaltet, dass die Pumpeinheit einen unsteten Druckverlauf derart erzeugen kann, dass der Druck zeitweise derart fällt, dass kurzzeitig gesaugt statt gepumpt wird. Hierdurch können beim jeweils nachfolgenden Pumpen besonders starke Druckstöße erzeugt werden.

Vorzugsweise weist das System ein Programm mit vom Bediener wählbaren Modi aus, bei denen eine der folgenden Werte oder Wertkombinationen als Ziel vorgegeben und vom Programm durch automatische Anpassung des Absaugens und Pumpens angestrebt werden:
(a) CO₂-Eliminierung: Ziel ist ein vom Bediener vorgegebenes Volumen CO₂, das pro Zeiteinheit eliminiert sein soll (sogenannte "Clearance"). Die Clearance kann automatisch durch Bestimmung des CO₂-Gehalts im Gasstrom im flexiblen Schlauch bestimmt werden.-Angestrebt wird das vorgegebene Ziel durch Veränderung des angesaugten und zurückgepumpten Gasvolumens, im Falle einer Kolbenpumpe durch Veränderung des Kolbenhubvolumens. Je kleiner der CO₂₋Gehalt im Gasstrom, desto größer muss das abgesaugte Gasvolumen pro Atemzug sein, um die gleiche Clearance zu erhalten.
(b) CO₂-Zielwert kombiniert mit Anpassungsgeschwindigkeit: Ziel ist ein bestimmter CO₂-Wert z.B. im Blut oder in der Ausatemluft, jedoch wird zusätzlich vom Bediener vorgegeben, mit welcher Geschwindigkeit oder innerhalb welcher Zeitspanne der genannte Zielwert erreicht werden soll. Das Ziel wird wie unter (a) beschrieben angestrebt, jedoch passt das System die Veränderung des abgesaugten Gasvolumens pro Atemzug in Abhängigkeit von der vorgegebenen Anpassungsgeschwindigkeit und der jeweils gemessenen Clearance an, erhöht also beispielsweise langsam das abgesaugte Gasvolumen pro Atemzug. Der CO₂-Wert kann hierbei entweder aus dem oben genannten Gasanalysesensor bestimmt werden oder über eine Schnittstelle von einem anderen System kontinuierlich oder intermittierend gelesen werden, beispielsweise von einem transcutanen CO₂-Sensor oder von einem Blutgasanalysegerät. Auch manuelle Eingabe durch den Anwender ist möglich.
(c) Atemfrequenz: Ziel ist eine einstellbare Atemfrequenz des Patienten, insbesondere eine niedrigere Atemfrequenz. Dies wird vom Programm durch Veränderung des angesaugten und zurückgepumpten Gasvolumens pro Atemzug angestrebt, indem zur Senkung der Atemfrequenz das Gasvolumen erhöht wird beziehungsweise zur Anregung der Atemfrequenz das Gasvolumen verringert wird.
(d) CO-Eliminierung: Ziel ist die schnellstmögliche Elimination des giftigen CO durch das therapeutische Prinzip der isokapnischen Hyperventilation. Das Programm ermittelt das für die jeweilige Atemfrequenz technisch maximal sinnvolle Gasvolumen für die Ansaugung und das Zurückpumpen. Ein einstellbarer CO₂-Zielwert wird nun primär durch die geregelte Zudosierung von CO₂ erreicht. Dies erfolgt über eines der Be- und Entlüftungsventile oder eine separate Dosiereinheit.

Diese Liste ist nicht abschließend. Das Programm kann weitere Modi vorsehen. Für jeden Modus ist außerdem eine Abschaltsequenz wählbar, bei der vorzugsweise eine automatische stufenweise Reduktion des angesaugten und zurückgepumpten Gasvolumens bis auf Null unter Berücksichtigung eines maximal tolerierten, einstellbaren Anstiegs der Atemfrequenz und/oder des CO₂₋Gehalts erfolgt, um eine Entwöhnung des Patienten zu unterstützen.

Vorzugsweise weist die Steuerung ein Programm für den Betrieb beim beatmeten Patienten auf, derart dass aufgrund von Daten des Sensors auf eine steigende oder fallende Synchronisation geschlossen und in der Folge mittels einer Schnittstelle ein Signal zur Anpassung der Beatmung durch das Beatmungsgerät, insbesondere bezüglich Atemfrequenz und Tidalvolumen, gegeben wird. Die Schnittstelle kann eine unmittelbare elektronische Schnittstelle zwischen dem System und dem Beatmungsgerät und/oder eine Bedienerschnittstelle, insbesondere ein Bildschirm oder eine Kontrollleuchte, sein. Bei einer Bedienerschnittstelle bleibt die tatsächliche Anpassung der Beatmung durch das Beatmungsgerät in der Verantwortung des Bedieners.

Die Offenbarung schlägt ein Patientenset vor zur Verwendung als Teil eines Systems wie zuvor beschrieben. Das Patientenset weist den flexiblen Schlauch, die Pumpeinheit und die Reservoireinheit und damit diejenigen Komponenten auf, die gegebenenfalls durch Keime des Patienten kontaminiert werden. Das Patientenset ist insbesondere als EinwegProdukt ausgeführt oder weist zumindest Teile auf, die Einwegprodukte sind. Das Patientenset kann entweder als unmontierter Bausatz oder als zumindest teilweise vormontiertes Kit ausgeführt sein. Auch können weitere Elemente, wie die oben beschriebene Verzweigung, Filter, Sensoren, Teile von Sensoren oder Anschlüsse von Sensoren, Teil des Patientensets sein.

Vorzugsweise weist das Patientenset eine Mess-Küvette für einen Gasanalyse-Sensor auf. Dies ermöglicht auf einfache Weise, dass ein Gasanalyse-Sensor, der aufwendig in der Herstellung ist, wiederholt, also für unterschiedliche Patienten, eingesetzt werden kann. Denkbar ist auch eine hygienische Abkopplung durch geeignete Filter.

In einer bevorzugten Ausführungsform weist das Patientenset eine Kolbenpumpe als Pumpeinheit und Reservoireinheit auf. Die Kolbenpumpe weist einen Kolben auf, der von einem Zylinderrohr umgeben ist. Der Kolben weist mindestens zwei Ringdichtungen zur Abdichtung gegenüber dem Zylinderrohr auf. Hierdurch kann ein Bereich, der von abgesaugter Luft gefüllt wird, von einem Bereich, der mit der Umgebungsluft in Kontakt tritt, konsequent getrennt werden. Dazu darf der Kolben gegenüber dem Zylinderrohr stets nur so weit verfahren werden, dass die Verfahrwege sich nicht überschneiden. Anders ausgedrückt gibt es keinen Innenabschnitt des Zylinderrohrs, der von beiden Ringdichtungen überstrichen wird. Hierdurch wird ein Übertritt von Keimen oder Partikeln in beide Richtungen vermieden. Um dies zu gewährleisten und gleichzeitig einen kompakten Aufbau zu erreichen, ist vorzugsweise der Abstand zwischen den Ringdichtungen größer als ein Drittel, insbesondere als die Hälfte, der Länge des Raums des Zylinderrohrs für den Kolben.

Erfindungsgemäß und für den Fall des beatmeten Patienten schlägt die Erfindung ein Patientenset vor, bei dem die Pumpeinheit und die Reservoireinheit durch eine Kolbenpumpe gebildet ist, und dass das Patientenset eine weitere Kolbenpumpe zur Ausbildung einer weiteren Pumpeinheit und einer weiteren Reservoireinheit aufweist. Die beiden Kolbenpumpen sind in fester Position zueinander und miteinander zu einem Kit verbunden, wobei dies nicht einstückig sein muss aber kann. Um sicher zu stellen, dass die Kolbenpumpen richtig in eine Einheit mit den Antrieben für die Kolbenpumpen eingesetzt werden, sieht die Erfindung insbesondere vor, dass das Kit mindestens bezüglich zweier Hauptebenen unsymmetrisch ist.

Dabei sind mit den Hauptebenen die sich aus den Kolbenlängsachsen ergebenden, orthogonalen Ebenen gemeint, wobei insbesondere die Kolbenlängsachsen in einer dieser Ebenen liegen. Anders ausgedrückt ist das Kit derart unsymmetrisch, dass mittels einer korrespondierenden Aufnahme ein vertauschtes Fügen der beiden Kolbenpumpen in die Aufnahme ausgeschlossen ist. Insbesondere ist das Kit bezüglich einer die Kolbenlängsachsen verbindenden Ebene und/oder einer hierzu senkrechten Ebene nicht spiegelsymmetrisch.

Für den Fall eines Systems mit nur einer Kolbenpumpe sieht die Offenbarung vorzugsweise ein Patientenset vor, derart dass die Pumpeinheit und die Reservoireinheit durch eine Kolbenpumpe gebildet ist, die mindestens bezüglich zweier Hauptebenen unsymmetrisch ist. Hierdurch wird erreicht, dass eine definierte Position und Orientierung relativ zur Antriebseinheit gewährleistet werden kann. Dies ist beispielsweise hilfreich, wenn an der Kolbenpumpe zusätzliche Elemente wie Kennzeichnungen angebracht sind.

Die Erfindung wird nachfolgend anhand eines Ausführungsbeispiels erläutert. Es zeigen:
- Figur 1: eine schematische Darstellung eines beispielhaften Systems in der Anwendung beim nicht-beatmeten Patienten;
- Figur 2: eine schematische Darstellung des erfindungsgemäßen Systems in der Anwendung beim beatmeten Patienten;
- Figur 3: eine perspektivische Darstellung einer Durchführungstülle feines beispielhaftenSystems in perspektivischer Darstellung;
- Figur 4: einen zweiten Konnektor eines beispielhaften Systems in perspektivischer Darstellung;
- Figur 4a: eine vergrößerte Darstellung entsprechend dem Ausschnitt I aus Figur 4; und
- Figur 5: ein beispielhaftes Kit aus zwei Kolbenpumpen mit deren Aufnahme und Antriebseinheiten in einer perspektivischen Darstellung.

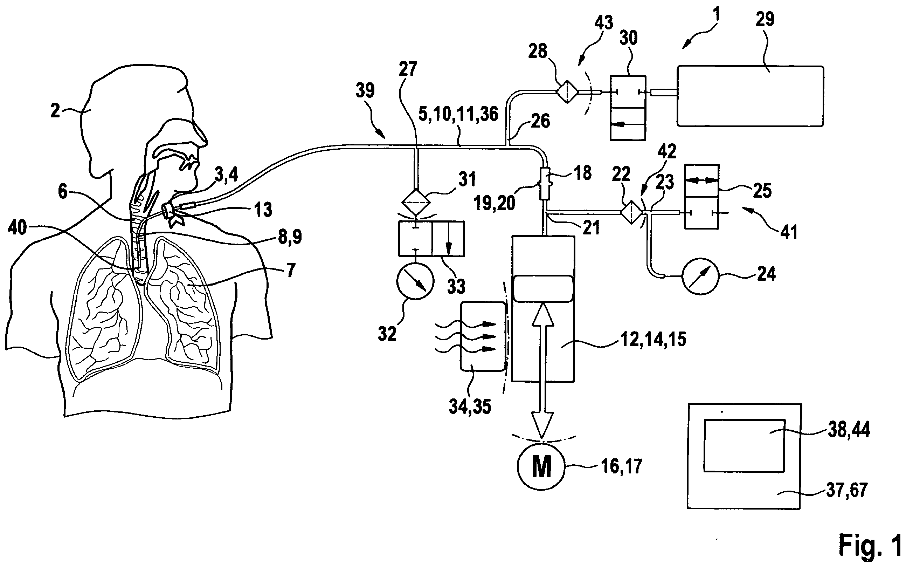

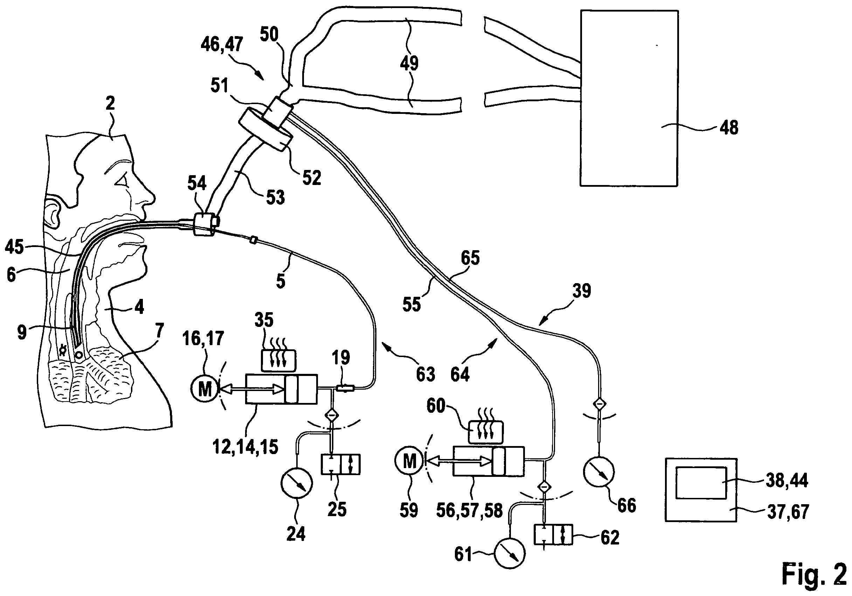

Figur 1 zeigt einen schematischen Uberblick über ein beispielhaftes System 1 in der Anwendung bei einem nicht-beatmeten Patienten 2. Durch eine mittels eines kleinen Eingriffs erzeugte Öffnung 3 im Bereich des Halses 4 des Patienten 2 ist ein flexibler Schlauch 5 des Systems 1 von außen in die Luftröhre 6 des Patienten 2 eingeführt worden und reicht bis nahe der Lunge 7 des Patienten 2. Der flexible Schlauch 5 besteht aus einem einen ersten Schlauchabschnitt 8 in Form eines Katheters 9 zum Einführen in die Luftröhre 6 und einen zweiten Schlauchabschnitt 10 in Form eines Verbindungsschlauchs 11 zur Verbindung des ersten Schlauchabschnitts mit einer Reservoireinheit 12 des Systems 1. Der Verbindungsschlauch 11 weist einen größeren Außenquerschnitt als der Katheter 9 auf. Im Bereich der Öffnung 3 ist der Katheter 9 von einer Durchführungstülle 13 umfasst und wird von dieser klemmend gegen eine Verschiebung längs des Katheters 9 gehalten. Die Durchführungstülle 13 ist in Figur 2 genauer dargestellt.

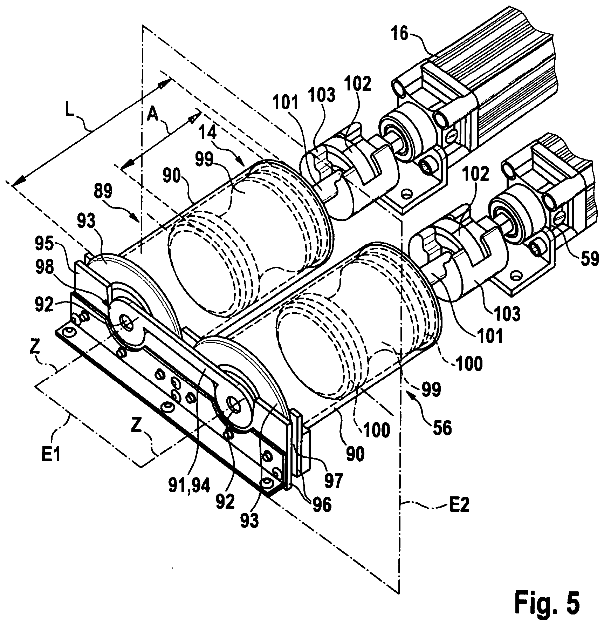

Die Reservoireinheit 12 ist als eine Kolbenpumpe 14 ausgebildet und ist somit gleichzeitig Pumpeinheit 15. Die Kolbenpumpe 14 wird von einem Linearmotor 16 als Antriebseinheit 17 angetrieben. Die Antriebseinheit 17 ist in Figur 1 nur symbolisch dargestellt. Im Folgenden wird anhand Figur 5 noch genauer auf die Antriebseinheit 17 eingegangen. Der Verbindungsschlauch 11 ist unterbrochen von einer Messküvette 18 für einen Gasanalyse-Sensor 19, hier ein CO₂-Sensor 20, der an der Messküvette 18 angebracht ist. Zwischen der Messküvette 18 und der Kolbenpumpe 14 ist der Verbindungsschlauch 11 außerdem durch einen ersten Abzweig 21 unterbrochen. Dieser erste Abzweig 21 ist wie auch die nachfolgenden Abzweige jeweils durch ein T-Stück oder Y-Stück gebildet. Der erste Abzweig 21 führt über einen ersten Filter 22, der wie auch die nachfolgend beschriebenen Filter eine hygienische Barriere bildet, zu einem zweiten Abzweig 23, an dessen einem Ausgang ein Hochdrucksensor 24 und dessen anderem Ausgang ein Be-und Entlüftungsventil 25 mit Ausgang zur Umgebung angeschlossen ist. Zwischen der Messküvette 18 und dem Katheter 9 ist der Verbindungsschlauch 11 nacheinander durch einen dritten Abzweig 26 und einen vierten Abzweig 27 unterbrochen. Der dritte Abzweig 26 führt über einen zweiten Filter 28 zu einem Druckbehälter 29, der über ein Dosierventil 30 fluidisch verbunden oder getrennt werden kann. Der Druckbehälter 29 enthält ein therapeutisches Gas, insbesondere Sauerstoff. Der vierte Abzweig 27 führt über einen dritten Filter 31 zu einem Feindrucksensor 32 mit einem Sensor-Schutzventil 33. Während der Hochdrucksensor 24 zum Messen von Drücken im Bereich von -750 mbar bis +750 mbar ausgelegt ist und damit der Kontrolle der Absaug- und Pumpdrücke der Kolbenpumpe 14 dient, ist der Feindrucksensor 32 zum Messen von Drücken im Bereich von -5mbar bis +5mbar ausgelegt und dient damit der Kontrolle der Atmung des Patienten 2, wenn die Kolbenpumpe 14 nicht arbeitet. Die Kolbenpumpe 14 ist durch ein thermoelektrisches Heizelement 34 als Temperiereinheit 35 beheizbar. Außerdem ist der flexible Schlauch 5 mit einer thermischen Isolierung 36 umhüllt.

Die Sensoren, also der Gasanalyse-Sensor 19, der Hochdrucksensor 24 und der Feindrucksensor 32, und die Aktoren, also der Linearmotor 16, das Heizelement 34, das Be- und Entlüftungsventil 25, das Dosierventil 30 und das Sensorschutzventil 33 sind jeweils über elektrische Leitungen, die der Übersicht halber nicht dargestellt sind, mit einer Steuerung 37 verbunden. Die Steuerung 37 umfasst insbesondere eine Ein- und Ausgabeeinheit in Form einer berührungsempfindlichen Anzeige 38, ein sogenannter "Touch-Screen", zur Bedienung durch einen Anwender.

Der Flexible Schlauch 5 und die Kolbenpumpe 14 sind zusammen mit dem ersten, dritten und vierten Abzweig 21, 26, 27 sowie den drei Filtern 22, 28, 31 und der Messküvette 18 ein Patientenset 39. Die Schnittstellen zu den restlichen Komponenten des Systems 1 sind jeweils durch Strichpunktlinien symbolisiert, wobei im Bereich der Filter 22, 28, 31 als Schnittstellen übliche Schlauchverbindungen in Form von Steck- oder Schraubverbindungen (nicht im Detail dargestellt) Verwendung finden. Um eine fehlerhafte Bedienung beim Anschließen auszuschließen sind die Schlauchverbindungen mechanisch kodiert, es passen also jeweils nur die richtigen Komponenten zueinander. Alternativ oder zusätzlich wäre außerdem eine elektronische Kodierung möglich derart, dass die Steuerung die richtige Verbindung automatisch erkennt. Dies könnte beispielsweise durch RFID-Elemente in den Schlauchverbindungen realisiert werden. Die Schnittstellen im Bereich der Kolbenpumpe 14 sind insbesondere mechanischer Art, wobei auch hier zusätzlich oder alternativ eine elektronische Überprüfung stattfinden kann. Das Patientenset 39 ist als Einwegprodukt ausgelegt, was nicht ausschließt, dass einzelne Komponenten für den wiederholten Gebrauch aufbereitet, also insbesondere gereinigt und sterilisiert, werden können.

Nachfolgend wird die Funktion des Systems 1 beschrieben: Ein offenes Ende 40 des Katheters 9 stellt eine fluidische Verbindung zum lungennahen Abschnitt der Luftröhre 6 her. Dadurch kann über den Feindrucksensor 32 die Atemtätigkeit des Patienten 2 bestimmt werden. In einem Normalbetrieb wird am Ende der Ausatemphase, also end-exspiratorisch, Gas mittels der Kolbenpumpe 14 abgesaugt und zum Ende der nächsten Einatemphase bzw. zu Beginn der nächsten Ausatemphase wieder zurück gepumpt.

Dieses Gas ist reich an CO₂ und kann daher als "verbrauchte Luft" bezeichnet werden. Durch das Absaugen und Zurückführen durch Pumpen wird bewirkt, dass bereits beim Absaugen frische Luft durch die freien oberen Atemwege (Nase und Mund) in die Luftröhre 6 des Patienten 2 strömt und bei dem danach stattfindenden Einatemvorgang nicht erst verbrauchte Luft in die Lunge gerät, sondern sofort sauerstoffreiche und CO₂-arme, also "frische Luft". Hierdurch wird die CO₂-Clearance in der Lunge sowie die O₂-Aufnahme deutlich verbessert. Das Zurückführen der verbrauchten Luft hat den Vorteil, dass die Balance des Energie- und Feuchtehaushalts der Atemwege nicht beeinträchtigt wird, da keine Feuchte und keine Energie entnommen oder zugeführt werden. Damit sich dieser Wasserdampf nicht als Kondensat absetzt und sich außerdem keine ungewünschte Kühlwirkung des Systems 1 auf den Patienten 2 ergibt, wird das abgepumpte Gas während der Zwischenspeicherung in der Kolbenpumpe mittels des Heizelements 34 warm gehalten und darüber hinaus ein Temperaturaustausch mit der Umgebung durch die thermische Isolierung 36 oder aktive Heizung des flexiblen Schlauchs 5 gering gehalten. Die Pumpgeschwindigkeit wird insbesondere mittels der Daten des Hochdrucksensors 24 geregelt. Sollte sich, beispielsweise aus den Druckverläufen beim Absaugen und Pumpen, für die Steuerung ergeben, dass der Katheter 9 verstopft ist, kann das System diesen automatisch freiblasen. Hierzu kann das Be- und Entlüftungsventil 25 beim Absaugen geöffnet werden, wodurch vorwiegend Gas aus der Umwelt angesaugt wird. Der erste Filter 22 sorgt dabei dafür, dass es zu keiner Kontamination mit Erregern aus der Umwelt kommt. Nach dem Absaugen wird das Be- und Entlüftungsventil 25 geschlossen und das Gas in der Kolbenpumpe 14 von dieser ausgepumpt. Insbesondere durch ein unstetiges, pulsartiges Pumpen kann erreicht werden, dass der Katheter 9 wieder freikommt. Das Be-und Entlüftungsventil 25 bildet somit eine zuschaltbare Öffnung 41 zur Umwelt und der erste Abzweig 21 zusammen mit dem zweiten Abzweig 23 eine Verzweigung 42 für die zuschaltbare Öffnung 41. Das Be- und Entlüftungsventil 25 kann auch dazu genutzt werden, das abgesaugte Gas ganz oder teilweise in die Umwelt auszublasen. Statt oder zusätzlich zu dem abgesaugten Gas kann dann über eine Öffnung des Dosierventils 30 aus dem Druckbehälter 29 ein therapeutisches Gas über den flexiblen Schlauch 5 zum Patienten 2 geleitet werden, wobei der Pumpvorgang hierzu auch zeitlich verschoben werden kann, insbesondere in die Phase der Einatmung des Patienten 2. Der dritte Abzweig 26 bildet zusammen mit dem Dosierventil 30 eine zuschaltbare Zuleitung 43, durch die statt oder zusätzlich zu dem rückgeführten Gas ein Volumenstrom eines weiteren Gases zu dem flexiblen Schlauch 5 geführt werden kann.

Die genannten Funktionen werden von der Steuerung 37 automatisch in Abhängigkeit von Eingaben des Bedieners einerseits und den Daten der Sensoren, also des Gasanalyse-Sensors 19, des Hochdrucksensors 24 und des Feindrucksensors 32, gesteuert. Die berührungsempfindliche Anzeige 38 dient dabei als eine Benutzerschnittstelle 44. Außerdem können mit der Steuerung 37 die im allgemeinen Teil beschriebenen Modi angewählt und betrieben werden. Gleiches gilt für die oben beschriebene Funktionalität der Steuerung 37 für den beatmeten Patienten, auf den in der Folge noch eingegangen wird. Zur Vermeidung von Wiederholungen wird auf diese Funktionen der Steuerung 37 nicht erneut eingegangen.

In der erfindungsgemäßen Figur 2 ist die Situation beim beatmeten Patienten 2 dargestellt, wobei für gleiche oder zumindest ähnliche Bauteile hier gleiche Bezugsziffern verwendet werden und zur Vermeidung von Wiederholungen im Folgenden lediglich auf die Unterschiede zur Situation in Figur 1 eingegangen wird. Im Gegensatz zu Figur 1 ist die zuschaltbare Zuleitung 43 und der Feindrucksensor 32 nicht dargestellt, da diese Bauteile beim beatmeten Patienten 2 zumeist keine Verwendung finden. Gleichwohl können sie dennoch vorhanden sein. Der Katheter 9 ist beim beatmeten Patienten nicht durch eine separate Öffnung im Hals 4 des Patienten 2 in die Luftrohre 6 geführt, sondern üblicherweise durch einen schlauchartigen Tubus 45, der Teil eines Leitungssystems 46 eines Beatmungssystems 47 ist.

Das Beatmungssystem 47 weist eine Zentraleinheit 48 mit Pumpe, Steuerung, Benutzerschnittstelle und dergleichen auf, worauf es hier nicht im Detail ankommt. Von dieser Zentraleinheit 48 gehen zwei Beatmungsschläuche 49 ab bis zu einem Y-Stück 50, an den sich nacheinander ein erster Konnektor 51, ein Filterelement 52 mit HME (Heat and Moisture Exchanger), eine schlauchartige Tubusverlängerung 53 und schließlich ein zweiter Konnektor 54 anschließt. An den zweiten Konnektor 54, der im Folgenden noch näher erläutert wird, ist der Tubus 45 angeschlossen. Das Beatmungssystem 47 arbeitet in bekannter Weise, das heißt über einen Beatmungsschlauch 49, den Tubus 45 und die dazwischen liegenden Bauteile wird Luft, Sauerstoff und/oder therapeutische Gase in die Lunge 7 des Patienten 2 gepumpt und im Wechsel dazu die verbrauchte Luft in umgekehrter Richtung, aber über den anderen Beatmungsschlauch 49 wieder abgeführt. Die Beatmungsschläuche 49 bilden zusammen mit dem Tubus 45 und den dazwischen liegenden Bauteilen das Leitungssystem 46 des Beatmungssystems 47. Natürlich sind hier auch Beatmungsgeräte denkbar, die nur einen Beatmungsschlauch aufweisen und beispielsweise ein patientennahes Ventil- oder Leckagesystem.

An den ersten Konnektor 51 ist ein weiterer flexibler Schlauch 55 angeschlossen. Dieser weitere flexible Schlauch 55 dient der Verbindung mit einer weiteren Kolbenpumpe 56, die eine weitere Pumpeinheit 57 und einer weitere Reservoireinheit 58 bildet. Die weitere Kolbenpumpe 56 wird über einen weiteren Linearmotor 59 angetrieben. Der Kolbenpumpe 56 ist eine weitere Temperiereinheit 60 zugeordnet und über eine Verzweigung und einen Filter ist ein weiterer Hochdrucksensor 61 sowie ein weiteres Be-und Entlüftungsventil 62 angeschlossen. Während der zuvor beschriebene flexible Schlauch 5 mit den daran angeschlossenen Elementen, wie der Kolbenpumpe 14, einen ersten Strang 63 bildet, bildet der weitere flexible Schlauch 55 mit den hieran angeschlossenen Elementen, wie der weiteren Kolbenpumpe 56, einen zweiten Strang 64. Der zweite Strang 64 weist keine Messküvette auf, was allerdings nicht ausgeschlossen ist, wenn in diesem Bereich beispielsweise die CO₂-Konzentration gemessen werden soll. Erfindungsgemäß sind der weitere flexible Schlauch 55 und die weitere Kolbenpumpe 56 Teil des Patientensets 39, wobei die Schnittstellen zu den restlichen Komponenten des Systems 1 analog zum ersten Strang 63 ausgebildet und wiederum jeweils durch Strichpunktlinien symbolisiert sind.

An den ersten Konnektor 51 ist außerdem als Teil des Patientensets 39 ein dritter flexibler Schlauch 65 angeschlossen, der über einen Filter mit einem Beatmungsdrucksensor 66 verbunden ist. Der Beatmungsdrucksensor 66 hat einen Messbereich bis +-100 mbar. Am Konnektor 51 sind der weitere Hochdrucksensor 61 und der Beatmungsdrucksensor 66 in Strömungsrichtung des Beatmungssystems 47 zueinander versetzt angeordnet. Zumindest solange die weitere Kolbenpumpe 56 nicht bewegt wird, kann über die Messung der Druckdifferenz durch die genannten Sensoren auf die Strömungsrichtung und Strömungsgeschwindigkeit im ersten Konnektor 51 und damit im Leitungssystem 46 des Beatmungssystems 47 geschlossen werden. Dies erlaubt es, das System 1 mit dem Beatmungssystem 47 zu synchronisieren.

Auch die Komponenten des zweiten Strangs 64 sowie der dritte Beatmungsdrucksensor 66 sind an die Steuerung 37 angeschlossen.

Die beiden Stränge 63, 64 unterstützen die Beatmung des Patienten 2, die im Wesentlichen durch das Beatmungssystem 47 erfolgt, indem mittels der beiden Kolbenpumpen 14, 56 jeweils im Wechsel abgepumpt und zurückgeführt wird. Dabei wird am Ende der Ausatmung verbrauchtes Atemgas vom ersten Strang 63 aus den Atemwegen 7 gesaugt, während im Wesentlichen gleichzeitig vom zweiten Strang 64 frisches Gas zurückgeführt wird. Tatsächlich beginnt das Zurückführen schon etwas früher, damit im Leitungssystem 46 des Beatmungssystems 47 keine Druckminderung erfolgt, die dem Beatmungssystem ein Einatmen der Lunge suggerieren würde. Für das leicht versetzte Arbeiten der Kolbenpumpen 14, 56 ist es entscheidend, dass diese nicht starr gekoppelt sondern durch je einen Linearmotor 16, 59 voneinander unabhängig angetrieben sind. Durch das Absaugen wird das verbrauchte CO₂-reiche Gas aus dem Totraum entfernt und durch das über den zweiten Strang zugeführte Frischgas ersetzt.

Das vom ersten Strang 63 abgesaugte Gas wird am Ende der folgenden Einatmung bzw. zum Beginn der folgenden Ausatmung wieder zurückgeführt, während im Wesentlichen gleichzeitig vom zweiten Strang 64 frisches Gas aus dem Beatmungssystem abgepumpt wird.

Alle oben für den nicht-beatmeten Patienten beschriebenen Elemente, wie die optionale nichtstetige Bewegung der Kolbenpumpe, sind auch bei dieser Ausbildung für den beatmeten Patienten erhalten, ebenso beispielsweise die oben beschriebenen Programme.

Das System 1 weist im Bereich der Steuerung 37 eine Kommunikationsschnittstelle 67 auf. Diese besteht sowohl aus einem Anschluss (nicht im Detail dargestellt) für eine Kabelverbindung, als auch aus einer WLAN- oder Bluetooth-Schnittstelle derart, dass sowohl kabelgebunden als auch drahtlos ein Austausch von Informationen mit Drittgeräten, externen Sensoren und/oder Fernüberwachungssystemen erfolgen kann. Insbesondere kann eine Kopplung mit dem Beatmungssystem 47 hergestellt werden.

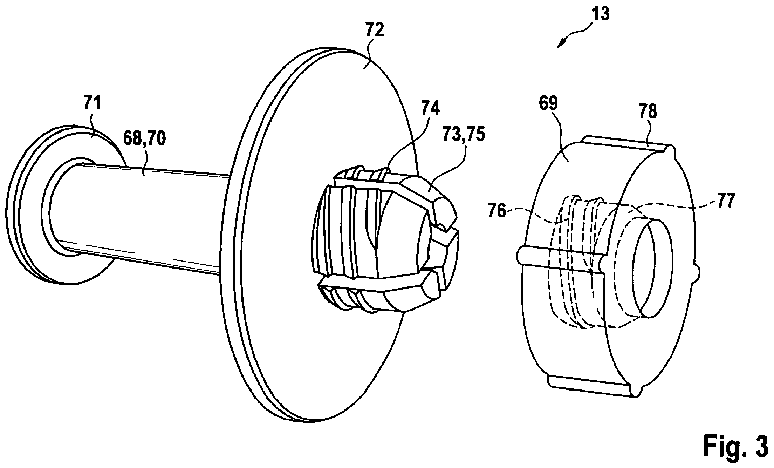

Im Folgenden wird auf einzelne Komponenten des Systems 1 näher eingegangen. Figur 3 zeigt die Durchführungstülle 13. Die Durchführungstülle 13 besteht aus zwei Teilen, zum einen einem Grundkörper 68 und zum anderen einer Klemmmanschette 69. Beide Teile sind im Wesentlichen rotationssymmetrisch. Der Grundkörper 68 weist ein Rohrstück 70 zwischen einem hinteren und einen vorderen Flansch 71, 72 auf, wobei der vordere Flansch 72 etwa doppelt so groß im Durchmesser ist wie der hintere flexible Flansch 71. Jenseits des vorderen Flanschs 72 geht das Rohrstück 70 in ein vierfach geschlitztes Klemmstück 73 über, das ein Außengewinde 74 und ein konisch zulaufendes Ende 75 aufweist. Die Klemmmanschette 69 ist hülsenartig und weist ein zum Außengewinde 74 passendes Innengewinde 76 auf, an das sich eine konische Durchmesserverjüngung 77 anschließt. Über den äußeren Umfang verteilt weist die Klemmmanschette 69 vier Längswulste 78 zur besseren Handhabung auf. Die Durchführungstülle 13 kann mit dem hinteren Flansch 71 voraus in die Öffnung 3 am Hals 4 des Patienten 2 eingebracht werden. Der hintere Flansch 71 verhindert, dass die Durchführungstülle 13 unbeabsichtigt aus der Öffnung 3 herausrutscht, und der vordere Flansch 72, dass sie weiter in die Öffnung 3 hineinrutscht. Im nächsten Schritt wird der Katheter 9 durch die Klemmmanschette 69 und den Grundkörper 68 hindurch in die Luftröhre 6 geschoben. Sobald der Katheter 9 weit genug eingeschoben ist, wird diese Position durch Klemmen des Katheters 9 in der Durchführungstülle 13 fixiert. Dazu wird die Klemmmanschette 69 auf das Klemmstück 73 geschraubt. Das konisch zulaufende Ende 75, die Durchmesserverjüngung 77 sowie die Schlitzung des Klemmstücks 73 bewirken zusammen, dass das Klemmstück 73 radial zusammen gedrückt wird, wodurch der Katheter 9 geklemmt wird.

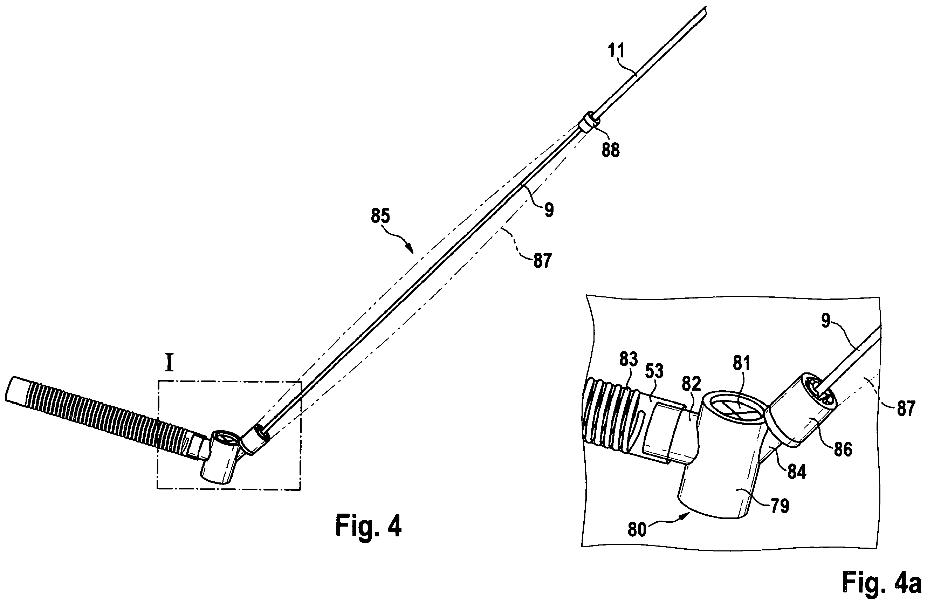

Figur 4 und Figur 4a zeigen den zweiten Konnektor 54. Dieser weist einen rohrartigen Konnektorgrundkörper 79, dessen erstes, offenes Ende einen Tubusanschluss 80 und dessen anderes Ende ein Entenschnabelventil 81 zum Einführen eines Einweg-Absaugkatheters (nicht dargestellt) zum Absaugen von Sekret aufweist. Zur einen radialen Seite weist der Konnektorgrundkörper 79 einen ersten Anschlussstutzen 82 für die Tubusverlängerung 53 auf, wobei die Figuren zeigen, dass die Tubusverlängerung 53 ein mittels einer Wendel 83 verstärkter Schlauch ist. Radial gegenüber dem Anschlussstutzen 82 weist der Konnektorgrundkörper 79 einen schräg vom Tubusanschluss 80 wegweisenden Katheteranschluss 84 auf. An den Katheteranschluss 84 ist der Katheter 9 mit einem Schutzhüllensystem 85 angeschlossen. Zu dem Schutzhüllensystem 85 gehört eine Klemmhülse 86, mit der durch axiale Verschiebung der Katheteranschluss 84 radial verengt werden kann, und eine sich an die Klemmhülse 86 anschließende Schutzhülle 87 in Form einer Schlauchfolie. Das gegenüberliegende Ende der Schlauchfolie endet an einem Verbindungsstück 88, das auch Teil des Schutzhüllensystems 85 ist und in dem der Katheter 9 fixiert ist. Gegenüberliegend ist der Verbindungsschlauch 11 befestigt, so dass das Verbindungsstück 88 die Verbindung zwischen dem Verbindungsschlauch 11 und dem Katheter 9 bildet. Die Figuren 4 und 4a zeigen den Zustand, in dem der Katheter vollständig aus dem Tubus 45 herausgezogen ist, beispielsweise um mit einem Absaugkatheter Sekret abzusaugen. Der Katheter 9 ist durch das Schutzhüllensystem 85 vor einer Kontamination geschützt und kann nach dem Absaugen wieder durch den zweiten Konnektor 54 hindurch in den Tubus 45 geführt werden, wobei die Schutzhülle 87 gestaucht wird. Durch Verschieben des Katheteranschlusses 84 wird der Katheter 9 geklemmt und so gegen unbeabsichtigte Verschiebung gesichert.

Ein derartiges Schutzhüllensystem kann auch in der Variante für nicht-beatmete Patienten im Bereich der Durchführungstülle 13 vorgesehen sein.

In der Figur 5 ist die Anordnung der Kolbenpumpe 14 und der weiteren Kolbenpumpe 56 als ein Kit 89 mit dem dazugehörigen Linearmotor 16 sowie dem weiteren Linearmotor 59 dargestellt. Die beiden Kolbenpumpen 14, 56 weisen jeweils ein transparentes Zylinderrohr 90 auf, das zum einen Ende hin durch einen gemeinsamen Zylinderkopf 91 verschlossen ist, bis auf eine Anschlussöffnung 92 für den flexiblen Schlauch 5 beziehungsweise den weiteren flexiblen Schlauch 55. Der Zylinderkopf 91 verbindet die beiden Zylinderrohre 90 und positioniert diese dadurch fest zueinander, wobei die Längsachsen Z der Zylinderrohre 90 parallel zueinander stehen und eine erste Hauptebene E1 des Kits 89 definieren. Der Zylinderkopf 91 weist jeweils koaxial zum Zylinderrohr 90 eine Kreisscheibe 93 und mit axialem Versatz dazwischen einen Verbindungssteg 94 auf, der jedoch radial versetzt zu den Längsachsen Z beziehungsweise parallel versetzt zur ersten Hauptebene E1 ist. Damit ist das Kit 89 nicht symmetrisch zur ersten Hauptebene E1. Gegenüber einer zweiten Hauptebene E2, die zur ersten Hauptebene E1 sowie zu den Längsachsen Z senkrecht steht, ist das Kit 89 ebenfalls nicht symmetrisch, da der Zylinderkopf 91 nur am einen Ende der Zylinderrohre 90 angeordnet ist. Dabei ist es unerheblich ist, wo entlang der Längsachse Z genau diese zweite Hauptebene E2 steht. Mit den Kreisscheiben 93 kann das Kit 89 in eine korrespondierend ausgebildete Aufnahme 95 eingesetzt werden und zwar entsprechend der fehlenden Symmetrie nur in der dargestellten, vorgesehenen Ausrichtung. Die Aufnahme 95 weist dazu für die Kreisscheiben 93 einen durch zwei beabstandete Bleche 96 gebildeten Schlitz 97 und für den Verbindungssteg 94 eine Ausnehmung 98 derart auf, dass ein vertauschtes Fügen der beiden Kolbenpumpen 14, 56 in die Aufnahme 95 ausgeschlossen ist. Im Zylinderrohr 90 ist jeweils ein Kolben 99 angeordnet. Die Kolben 99 weisen jeweils an ihren Enden eine umlaufende Ringdichtung 100 auf. Ein Abstand A der Ringdichtungen 100 entspricht etwa der Hälfte einer Länge des Raums des jeweiligen Zylinderrohrs 90 für den Kolben 99. Hierdurch wird gewährleistet, dass die von den Ringdichtungen 100 überstrichenen Längsbereiche sich nicht überschneiden und es in keiner Richtung zu einer Kontamination zwischen dem Inneren der Kolbenpumpen 14, 56 und der Umgebung kommt. Die Kolben 99 sind jeweils an ihren dem Zylinderkopf 91 abgewandten Enden durch eine Kolbenstange 101 mit einem rotationssymmetrischen, scheibenförmigen Griffstück 102 verlängert. Die Griffstücke 102 sind jeweils in eine Klaue 103 radial eingeschnappt, die mit dem jeweiligen Linearmotor 16, 59 verbunden ist. Die Klaue 103 weist dazu eine mit dem Griffstück 102 korrespondierende Gegenkontur auf. Die Gegenkontur ist so gestaltet, dass sie das Griffstück 102 über etwas mehr als den halben Umfang umgreift, wodurch dieses eingeschnappt werden kann. Damit sind die die Pumpeinheiten 15, 57 bildenden Kolbenpumpen 14, 56 werkzeuglos koppelbar und trennbar von den die Antriebseinheiten bildenden Linearmotoren 16, 59.

### Bezuqszeichenliste

- 1: System
- 2: Patient
- 3: Öffnung
- 4: Hals
- 5: Flexibler Schlauch
- 6: Luftröhre
- 7: Lunge
- 8: Erster Schlauchabschnitt
- 9: Katheter
- 10: Zweiter Schlauchabschnitt
- 11: Verbindungsschlauch
- 12: Reservoireinheit
- 13: Durchführungstülle
- 14: Kolbenpumpe
- 15: Pumpeinheit
- 16: Linearmotor
- 17: Antriebseinheit
- 18: Messküvette
- 19: Gasanalyse-Sensor
- 20: CO₂-Sensor
- 21: Erster Abzweig
- 22: Erster Filter
- 23: Zweiter Abzweig
- 24: Hochdrucksensor
- 25: Be- und Entlüftungsventil
- 26: Dritter Abzweig
- 27: Vierter Abzweig
- 28: Zweiter Filter
- 29: Druckbehälter
- 30: Dosierventil
- 31: Dritter Filter
- 32: Feindrucksensor
- 33: Sensor-Schutzventil
- 34: Heizelement
- 35: Temperiereinheit
- 36: Thermische Isolierung
- 37: Steuerung
- 38: Berührungsempfindliche Anzeige
- 39: Patientenset
- 40: Offenes Ende des flexiblen Schlauchs 5
- 41: Zuschaltbare Öffnung zur Umwelt
- 42: Verzweigung für die zuschaltbare Öffnung 41
- 43: Zuschaltbare Zuleitung
- 44: Benutzerschnittstelle
- 45: Tubus
- 46: Leitungssystem des Beatmungssystems 47
- 47: Beatmungssystem
- 48: Zentraleinheit des Beatmungssystems 47
- 49: Beatmungsschlauch
- 50: Y-Stück
- 51: Erster Konnektor
- 52: Filterelement
- 53: Tubusverlängerung
- 54: Zweiter Konnektor
- 55: Weiterer flexibler Schlauch
- 56: Weitere Kolbenpumpe
- 57: Weitere Pumpeinheit
- 58: Weitere Reservoireinheit
- 59: Weiterer Linearmotor
- 60: Weitere Temperiereinheit
- 61: Weiterer Hochdrucksensor
- 62: Weiteres Be- und Entlüftungsventil
- 63: Erster Strang
- 64: Zweiter Strang
- 65: Dritter flexibler Schlauch
- 66: Beatmungsdrucksensor
- 67: Kommunikationsschnittstelle
- 68: Grundkörper der Durchführungstülle 13
- 69: Klemmmanschette der Durchführungstülle 13
- 70: Rohrstück des Grundkörpers 68
- 71: Hinterer Flansch des Grundkörpers 68
- 72: Vorderer Flansch des Grundkörpers 68
- 73: Klemmstück des Grundkörpers 68
- 74: Außengewinde des Klemmstücks 73
- 75: Ende des Klemmstücks 73
- 76: Innengewinde der Klemmmanschette 69
- 77: Durchmesserverjüngung der Klemmmanschette 69
- 78: Längswulst der Klemmmanschette 69
- 79: Konnektorgrundkörper
- 80: Tubusanschluss
- 81: Entenschnabelventil
- 82: Anschlussstutzen für die Tubusverlängerung 53
- 83: Wendel
- 84: Katheteranschluss
- 85: Schutzhüllensystem
- 86: Klemmhülse
- 87: Schutzhülle
- 88: Verbindungsstück
- 89: Kit
- 90: Zylinderrohr
- 91: Zylinderkopf
- 92: Anschlussöffnung
- 93: Kreisscheibe
- 94: Verbindungssteg
- 95: Aufnahme
- 96: Blech
- 97: Schlitz
- 98: Ausnehmung
- 99: Kolben
- 100: Ringdichtung
- 101: Kolbenstange
- 102: Griffstück
- 103: Klaue
- A: Abstand zwischen den Ringdichtungen 100
- E1: Erste Hauptebene des Kits 89
- E2: Erste Hauptebene des Kits 89
- L: Länge des Raums des Zylinderrohrs 90 für den Kolben 99
- Z: Längsachse des Zylinderrohrs 90

## Patentansprüche

1. System (1) zur Unterstützung des pulmonalen Gasaustauschs bei Patienten (2) und zur Kopplung an ein Beatmungssystem (47) mit einem in die Luftröhre (6) eines Patienten (2) einführbaren flexiblen Schlauch (5), einer Pumpeinheit (15), einer Reservoireinheit (12) und einer Steuerung (37) die derart konfiguriert ist, dass über den flexiblen Schlauch (5) mittels der Pumpeinheit (15) eine exspiratorische, insbesondere end-exspiratorische, Absaugung und eine inspiratorische, insbesondere end-inspiratorische, oder beginnend-exspiratorische Rückführung des abgesaugten Gases einstellbar ist, wobei das System (1) einen Sensor (19, 24, 32) aufweist, und wobei das System (1) zur Absaugung und Rückführung von Gas aus dem Leitungssystem (46) eines Beatmungssystems (47) einen weiteren flexiblen Schlauch (55), eine weitere Pumpeinheit (57) und eine weitere Reservoireinheit (58) aufweist, **dadurch gekennzeichnet, dass** die Pumpeinheit (15) und die weitere Pumpeinheit (57) unabhängig voneinander durch die Steuerung (37) steuerbar sind.

2. System nach Anspruch 1, **dadurch gekennzeichnet, dass** das System (1) als Sensor einen Hochdrucksensor (24) aufweist, der derart gestaltet und angeordnet ist, dass er zur Erfassung der durch die Pumpeinheit (15) erzeugten Druckschwankungen geeignet ist.

3. System nach einem der vorstehenden Ansprüche, **dadurch gekennzeichnet, dass** der flexible Schlauch (5) einen ersten Schlauchabschnitt (8) zum Einführen in die Luftröhre (6) und einen zweiten Schlauchabschnitt (10) zur Verbindung des ersten Schlauchabschnitts (8) mit der Reservoireinheit (12) mit einem größeren Außenquerschnitt aufweist.

4. System nach einem der vorstehenden Ansprüche, **dadurch gekennzeichnet, dass** das System als Sensor einen Gasanalyse-Sensor (19), insbesondere einen CO₂-Sensor (20), aufweist, der bezüglich der Strömung des vom System (1) geführten Gases zwischen einem offenen Ende (40) des flexiblen Schlauchs (5) und der Reservoireinheit (12) angeordnet ist.

5. System nach einem der vorstehenden Ansprüche, **dadurch gekennzeichnet, dass** das System (1) eine Verzweigung (42) mit einer zuschaltbaren Öffnung (41) zur Umwelt, dem Beatmungskreislauf oder einer externen Gasquelle aufweist, derart dass mittels der Pumpeinheit (15) hieraus Gas angesaugt werden kann bzw. das Einströmen von Gas ermöglicht wird, vorzugsweise zum Freiblasen des flexiblen Schlauchs (5) im Falle einer Verstopfung.

6. System nach einem der vorstehenden Ansprüche, **dadurch gekennzeichnet, dass** die Pumpeinheit (15) planmäßig durch den Anwender werkzeuglos koppelbar und trennbar ist von einer Antriebseinheit (17) für die Pumpeinheit (15).

7. System nach einem der vorstehenden Ansprüche, **dadurch gekennzeichnet, dass** das System (1) eine Durchführungstülle (13) aufweist zur Durchführung und Fixierung des flexiblen Schlauchs (5) in eine operativ geschaffene Öffnung (3) in die Luftröhre (6) und zum Offenhalten dieser Öffnung (3).

8. System nach einem der vorstehenden Ansprüche, **dadurch gekennzeichnet, dass** die Steuerung (37) derart gestaltet ist, dass in Abhängigkeit von Daten des Sensors und/oder Eingaben über eine Benutzerschnittstelle (44) eine Regelung und/oder Ausgabe mit mindestens einer der folgenden Ausgangsgrößen erfolgt: Zeitpunkt des Pumpen/Absaugens, Geschwindigkeitsprofil des Pumpen/Absaugens, Volumen des Pumpen/Absaugens.

9. System nach einem der vorstehenden Ansprüche, **dadurch gekennzeichnet, dass** das System (1) derart gestaltet ist, dass durch die Pumpeinheit (15) ein Gasstrom mit unstetem, insbesondere gepulstem, Druckverlauf am patientenseitigen Endes des flexiblen Schlauchs (5) erzeugbar ist.

10. Verfahren zur Herstellung eines Systems (1) nach einem der vorstehenden Ansprüche unter Verwendung eines Patientensets (39) als Teil des Systems (1) **dadurch gekennzeichnet, dass** das Patientenset (39) den flexiblen Schlauch (5), die Pumpeinheit (15) und die Reservoireinheit (12) aufweist, dass die Pumpeinheit (15) und die Reservoireinheit (12) durch eine Kolbenpumpe (14) gebildet ist, und dass das Patientenset (39) einen weiteren flexiblen Schlauch (55) und eine weitere Kolbenpumpe (56) zur Ausbildung der weiteren Pumpeinheit (57) und der weiteren Reservoireinheit (58) aufweist.

11. Verfahren nach Anspruch 10, **dadurch gekennzeichnet, dass** das Patientenset (39) eine Mess-Küvette (18) für einen Gasanalyse-Sensor (19) aufweist.

12. Verfahren nach Anspruch 11, **dadurch gekennzeichnet, dass** die Pumpeinheit (15) und die Reservoireinheit (12) durch eine Kolbenpumpe (14) mit einem Kolben (99), der von einem Zylinderrohr (90) umgeben ist, gebildet ist, und dass der Kolben (99) mindestens zwei Ringdichtungen (100) zur Abdichtung gegenüber dem Zylinderrohr (90) aufweist.

13. Verfahren nach Anspruch 12, **dadurch gekennzeichnet,** die Kolbenpumpe (14) und die weitere Kolbenpumpe (56) in fester Position zueinander und miteinander als Kit (89) verbunden sind, und dass das Kit (89) mindestens bezüglich zweier Hauptebenen (E1, E2) unsymmetrisch ist.

## Claims

1. System (1) for assisting pulmonary gas exchange in patients (2) and for coupling to a respiration system (47) with a flexible tube (5) that can be inserted into the trachea (6) of a patient (2), a pumping unit (15), a reservoir unit and a controller (37) which is configured such that an expiratory aspiration, in particular end-expiratory aspiration, and an inspiratory recirculation, in particular end-inspiratory or starting-expiratory recirculation, of the aspirated gas can be set via the flexible tube (5) by means of the pump unit (15), wherein the system (1) comprises a sensor (19, 24, 32) and wherein the system (1) comprises a further flexible tube (55), a further pump unit (57) and a further reservoir unit (58) for aspirating and recirculating gas from the line system (46) of a respiration system (47), **characterized in that** the pump unit (15) and the further pump unit (57) can be controlled independently of each other by the controller (37).

2. System according to claim 1, **characterized in that** the system (1) as sensor is a high-pressure sensor (24) which is designed and arranged such as to be suitable for detecting the pressure fluctuations produced by the pump unit (15).

3. System according to any one of the preceding claims, **characterized in that** the flexible tube (5) has a first tube portion (8) for insertion into the trachea (6) and a second tube portion (10) for connecting the first tube portion (8) to the reservoir unit (12) with a larger outer section.

4. System according to any one of the preceding claims, **characterized in that** the sensor of the system is a gas analysis sensor (19), in particular a CO₂ sensor (20), which in relation to the flow of the gas guided by the system (1) is arranged between an open end (40) of the flexible tube (5) and the reservoir unit (12).

5. System according to any one of the preceding claims, **characterized in that** the system (1) comprises a branch (42) with an opening (41) that can be activated towards the environment, the respiration circuit or an external gas source, such that gas can be aspired therefrom by means of the pump unit (15) or an inflow of gas is made possible, preferably for the purpose of blowing free the flexible tube (5) in case it is clogged.

6. System according to any one of the preceding claims, **characterized in that** the pump unit (15) can be coupled to and separated from a drive unit (17) for the pump unit (15) by the user as planned, without any need for tools.

7. System according to any one of the preceding claims, **characterized in that** the system (1) has a grommet (13) for passing and fixing the flexible tube (5) in an operatively created opening (3) into the trachea (6) and for holding said opening (3) open.

8. System according to any one of the preceding claims, **characterized in that** the controller (37) is designed such that, as a function of data of the sensor and/or inputs, a regulation and/or output with at least one of the following output variables takes place via a user interface (44): Time of pumping/aspiration, speed profile of pumping/aspiration, volume of pumping/aspiration.

9. System according to any one of the preceding claims, **characterized in that** the system (1) is designed such that the pump unit (15) can produce a gas flow with an unsteady, in particular pulsed, pressure profile on the patient's end of the flexible tube (5).

10. Method for producing a system (1) according to any one of the preceding claims using a patient set (39) as part of the system (1), **characterized in that** the patient set (39) comprises the flexible tube (5), the pump unit (15), and the reservoir unit (12), that the pump unit (15) and the reservoir unit (12) is formed by a piston pump (14), and that the patient set (39) comprises a further flexible tube (55) and a further piston pump (56) to form the further pump unit (57) and the further reservoir unit (58).

11. Method according to claim 10, **characterized in that** the patient set is a measurement cuvette (18) for a gas analysis sensor (19).

12. Method according to claim 11, **characterized in that** the pump unit (15) and the reservoir unit (12) is formed by a piston pump (14) with a piston (99), which is enclosed by a cylinder pipe (90), and that the piston (99) has at least two ring seals (100) for sealing with respect to the cylinder pipe (90).

13. Method according to claim 12, **characterized in that** the piston pump (14) and the further piston pump (56) are connected in a fixed position relative to one another and to each other as a kit (89), and that the kit (89) is asymmetrical at least with respect to two main planes (E1, E2).

## Revendications

1. Système (1) destiné à assister l'échange gazeux pulmonaire chez des patients (2) et à s'accoupler à un système respiratoire (47) présentant un tuyau flexible (5) pouvant être introduit dans la trachée (6) d'un patient (2), une unité formant pompe (15), une unité formant réservoir (12) et un dispositif de commande (37) conçu de manière telle qu'une aspiration d'expiration, en particulier en fin d'expiration, et un retour d'inspiration, en particulier en fin d'inspiration ou en début d'expiration, du gaz aspiré peuvent être réglés par l'intermédiaire du tuyau flexible (5) au moyen de l'unité formant pompe (15), le système (1) présentant un capteur (19, 24, 32) et le système (1) présentant, pour l'aspiration et le retour de gaz provenant du système de tubulures (46) d'un système respiratoire (47), un autre tuyau flexible (55), une autre unité formant pompe (57) et une autre unité formant réservoir (58), **caractérisé en ce que** l'unité formant pompe (15) et l'autre unité formant pompe (57) peuvent être commandées indépendamment l'une de l'autre par le dispositif de commande (37).

2. Système selon la revendication 1, **caractérisé en ce que** le système (1) présente, comme capteur, un capteur haute pression (24), qui est conçu et disposé de manière telle qu'il convient pour la détection des oscillations de pression générées par l'unité formant pompe (15).

3. Système selon l'une quelconque des revendications précédentes, **caractérisé en ce que** le tuyau flexible (5) présente une première section de tuyau (8) destinée à l'introduction dans la trachée (6) et une deuxième section de tuyau (10) pour le raccordement de la première section de tuyau (8) à l'unité formant réservoir (12) présentant une section transversale externe plus grande.

4. Système selon l'une quelconque des revendications précédentes, **caractérisé en ce que** le système présente, comme capteur, un capteur d'analyse de gaz (19), en particulier un capteur de CO₂ (20), qui est disposé, par rapport à l'écoulement du gaz guidé par le système (1), entre une extrémité ouverte (40) du tuyau flexible (5) et l'unité formant réservoir (12).

5. Système selon l'une quelconque des revendications précédentes, **caractérisé en ce que** le système (1) présente une dérivation (42) pourvue d'une ouverture commutable (41) vers l'environnement, le circuit respiratoire ou une source gazeuse externe, de manière telle que du gaz peut être aspiré depuis celle-ci ou que l'admission de gaz est permise au moyen de l'unité formant pompe (15), de préférence pour libérer le tuyau flexible (5) par soufflage en cas de bouchage.

6. Système selon l'une quelconque des revendications précédentes, **caractérisé en ce que** l'unité formant pompe (15) peut être accouplée à une unité d'entraînement (17) pour l'unité formant pompe (15) et peut en être séparée de manière méthodique par l'utilisateur, sans outil.

7. Système selon l'une quelconque des revendications précédentes, **caractérisé en ce que** le système (1) présente un embout de passage (13) pour le passage et la fixation du tuyau flexible (5) dans une ouverture (3) réalisée de manière opératoire dans la trachée (6) et pour maintenir cette ouverture (3) ouverte.

8. Système selon l'une quelconque des revendications précédentes, **caractérisé en ce que** le dispositif de commande (37) est conçu de manière telle qu'en fonction des données du capteur et/ou des entrées par l'intermédiaire d'une interface utilisateur (44), une régulation et/ou une émission comprenant au moins l'une des grandeurs de sortie suivantes a lieu : moment du pompage/de l'aspiration, profil de vitesse du pompage/de l'aspiration, volume du pompage/de l'aspiration.

9. Système selon l'une quelconque des revendications précédentes, **caractérisé en ce que** le système (1) est conçu de manière telle que l'unité formant pompe (15) peut générer un flux gazeux présentant une allure de pression inconstante, en particulier pulsée, au niveau de l'extrémité côté patient du tuyau flexible (5).

10. Procédé pour la fabrication d'un système (1) selon l'une quelconque des revendications précédentes à l'aide d'un set de patient (39) comme partie du système (1), **caractérisé en ce que** le set de patient (39) présente le tuyau flexible (5), l'unité formant pompe (15) et l'unité formant réservoir (12), **en ce que** l'unité formant pompe (15) et l'unité formant réservoir (12) sont formées par une pompe à piston (14) et **en ce que** le set de patient (39) présente un autre tuyau flexible (55) et une autre pompe à piston (56) pour former l'autre unité formant pompe (57) et l'autre unité formant réservoir (58).

11. Procédé selon la revendication 10, **caractérisé en ce que** le set de patient présente une cuvette de mesure (18) pour un capteur d'analyse de gaz (19).

12. Procédé selon la revendication 11, **caractérisé en ce que** l'unité formant pompe (15) et l'unité formant réservoir (12) sont formées par une pompe à piston (14) pourvue d'un piston (99) entouré par un tube cylindrique (90) et **en ce que** le piston (99) présente au moins deux joints annulaires (100) pour la fermeture étanche par rapport au tube cylindrique (90).

13. Procédé selon la revendication 12, **caractérisé en ce que** la pompe à piston (14) et l'autre pompe à piston (56) sont reliées en position fixe l'une par rapport à l'autre et l'une à l'autre sous forme de kit (89) et **en ce que** le kit (89) est asymétrique au moins par rapport à deux plans principaux (E1, E2).
